# EUROPEAN PATENT APPLICATION

(11) **EP 3 845 261 A1**
(43) Date of publication of application: **07.07.2021**
(21) Application number: 19870937.0
(22) Date of filing: 11.10.2019
(51) Int. Cl.: A61M 21/02, A61M 21/00

(54) **AIR FLOW DISCHARGING DEVICE, SLEEPER SUPPORTING DEVICE, AND TIME NOTIFICATION DEVICE**

(30) Priority: 12.10.2018 JP 2018193434
(71) Applicant: DAIKIN INDUSTRIES, LTD., Osaka 530-8323 (JP)
(72) Inventor: NAKAGAWA, Ryouichi, Osaka-shi, Osaka 530-8323 (JP); MURAI, Yuuichi, Osaka-shi, Osaka 530-8323 (JP); SANAGI, Takaaki, Osaka-shi, Osaka 530-8323 (JP); KOSUGE, Hiroyasu, Osaka-shi, Osaka 530-8323 (JP)
(74) Representative: Goddar, Heinz J.
(86) International application number: PCT/JP2019/040311
(87) International publication number: WO 2020/075854

(57) **Abstract**

A discharging port (34) is provided, and an air flow is intermittently discharged through the discharging port (34) so that a periodical air flow for exerting a predetermined effect hits a target person (T).

## Description

### Technical Field

The Present disclosure relates to an air flow discharging device, a sleeper supporting device, and a time notification device.

### Background Art

A vibrating sleep-induction device described in PTL 1 includes a vibrator to be provided at bedding. The vibrating sleep-induction device applies vibrations to a sleeper from the vibrator so as to synchronize with the pulse of the sleeper on the bedding. These vibrations exert a sleep inducing effect on the sleeper.

### Citation List

### Patent Literature

PTL 1: Japanese Unexamined Patent Application Publication No. 3-70573

### Summary of Invention

### Technical Problem

In the device in PTL 1, vibrations are applied to bedding by the vibrator, and therefore, theses vibrations are easily transmitted to the surroundings. There is thus a likelihood of the vibrations being transmitted to a different target person that differs from a target person on which a predetermined effect is intended to be exerted.

An object of the present disclosure is to cause a device that exerts a predetermined effect on a target person to suppress vibrations from being transmitted to a different target person.

### Solution to Problem

A first aspect is an air flow discharging device including a discharging port (34) and configured to intermittently discharge an air flow through the discharging port (34) so that an air flow for exerting a predetermined effect hits a target person (T) periodically.

In the first aspect, an air flow is intermittently discharged through the discharging port (34), and the air flow periodically hits the target person (T) to exert a predetermined effect. Consequently, it is possible to suppress vibrations from being transmitted to the periphery of the target person (T).

A second aspect is a sleeper supporting device including the air flow discharging device (30) according to the first aspect in which the air flow discharging device (30) is configured to intermittently discharge an air flow through the discharging port (34) so that an air flow hits a sleeper (T) that is the target person at a predetermined rhythm.

In the second aspect, an air flow discharged through the discharging port (34) and having a predetermined rhythm hits the sleeper (T) to obtain a predetermined effect, and thus, it is possible to suppress vibrations from being transmitted to the periphery of the sleeper (T).

A third aspect is the sleeper supporting device according to the second aspect in which the air flow discharging device (30) is a vortex ring generator that has an extruding mechanism (40) configured to carry air, and that is configured such that air extruded by the extruding mechanism (40) is discharged as a vortex-ring-shaped air flow through the discharging port (34).

In the third aspect, it is possible to cause a vortex-ring-shaped air flow having high directivity to hit the sleeper (T).

A fourth aspect is the sleeper supporting device according to the third aspect in which the extruding mechanism (40) has a driver (41) and a vibrating plate (42) configured to be driven by the driver (41).

In the fourth aspect, it is possible to discharge a vortex-ring-shaped air flow at a comparatively short period by vibrating the vibrating plate (42) with the driver (41).

A fifth aspect is the sleeper supporting device according to any one of the second to fourth aspects further including an air flow regulator (40, 45) configured to change the air flow.

The fifth aspect can cause air flows in various states to hit the sleeper (T).

A sixth aspect is the sleeper supporting device according to the fifth aspect in which the air flow regulator (40) is configured to regulate a period of discharging an air flow.

In the sixth aspect, it is possible to regulate an interval of an air flow hitting the sleeper (T).

A seventh aspect is the sleeper supporting device according to the fifth or sixth aspect in which the air flow regulator (40) is configured to regulate a velocity of an air flow.

In the seventh aspect, it is possible to regulate an air pressure that hits the sleeper (T).

An eighth aspect is the sleeper supporting device according to any one of the fifth to seventh aspect in which the air flow regulator (45) is configured to regulate a direction of an air flow.

In the eighth aspect, it becomes easy to cause an air flow to hit the sleeper (T) or a predetermined part of the sleeper (T).

A ninth aspect is the sleeper supporting device according to any one of the fifth to eighth aspects further including a biological information acquisition unit (22) configured to acquire biological information of the sleeper (T), and in which the air flow regulator (40) is configured to change the air flow based on biological information acquired by the biological information acquisition unit (22) .

In the ninth aspect, it is possible to cause an air flow in accordance with biological information of the sleeper (T) to hit the sleeper (T).

A tenth aspect is the sleeper supporting device according to any one of the fifth to ninth aspects further including a positional information acquisition unit (22) configured to acquire positional information of the sleeper (T), and in which the air flow regulator (40) is configured to change the air flow based on positional information of the sleeper (T) acquired by the positional information acquisition unit (22).

In the tenth aspect, it is possible to cause an air flow in accordance with positional information of the sleeper (T) to hit the sleeper (T).

An eleventh aspect is the sleeper supporting device according to any one of the second to tenth aspects in which the air flow discharging device (30) is configured to discharge the air flow during a period of time in which the sleeper (T) is caused to sleep.

A twelves aspect is the sleeper supporting device according to any one of the second to eleventh aspects in which the air flow discharging device (30) is configured to discharge the air flow at a time of wake-up during a period of time in which the sleeper (T) is caused to be awakened.

A thirteenth aspect is the sleeper supporting device according to any one of the second to twelves aspects further including an illumination device (50) configured to illuminate a target space (S) in which the sleeper is present, and a control unit (70) configured to control the illumination device (50) in conjunction with the air flow discharging device (30).

In the thirteenth aspect, it is possible to exert a predetermined effect relating to sleep on the sleeper (T) by the control of the illumination device (50).

A fourteenth aspect is the sleeper supporting device according to any one of the second to thirteenth aspects further including a sound generator (90) configured to transmit a sound to a target space (S) in which the sleeper (T) is present, and a control unit (70) configured to control the sound generator (90) in conjunction with the air flow discharging device (30).

In the fourteenth aspect, it is possible to exert a predetermined effect relating sleep on the sleeper (T) by the control of the sound generator (90).

A fifteenth aspect is the sleeper supporting device according to any one of the second to fourteenth aspects in which the air flow discharging device (30) includes a component supplier (36) configured to add a predetermined discharge component to an air flow.

In the fifteenth aspect, it is possible to supply an air flow containing a predetermined discharge component to the sleeper (T).

A sixteenth aspect is the sleeper supporting device according to any one of the second to fifteenth aspects in which the air flow discharging device (30) is configured to be installed at a ceiling (C).

In the sixteenth aspect, it is possible to cause an air flow discharged from the side of the ceiling (C) to hit the sleeper (T).

A seventeenth aspect is a time notification device including the air flow discharging device (30) according to the first aspect and in which the air flow discharging device (30) is configured to perform a first operation of discharging an air flow through the discharging port (34) so that an air flow for notifying the target person (T) of a time hits the target person (T) periodically.

In the seventeenth aspect, an air flow that periodically hits the target person (T) enables the target person (T) to know a time. The time mentioned here includes a time point and a time that has elapsed.

An eighteenth aspect is the time notification device according to the seventeenth aspect in which the air flow discharging device (30) is configured to perform the first operation at every set time interval or every set time point.

In the eighteenth aspect, the target person (T) can know that a set time has elapsed or that a set time point has come.

A nineteenth aspect is the time notification device according to the seventeenth or eighteenth aspect in which the air flow discharging device (30) is configured to discharge air flows of a number corresponding to a current time point through the discharging port (34) in the first operation.

In the nineteenth aspect, the target person (T) can know a current time point on the basis of the number of air flows discharged through the discharging port (34) in the first operation.

A twentieth aspect is the time notification device according to any one of the seventeenth to nineteenth aspects in which the air flow discharging device (30) is a vortex ring generator that has an extruding mechanism (40) configured to carry air, and that is configured such that air extruded by the extruding mechanism (40) is discharged as a vortex-ring-shaped air flow through the discharging port (34).

In the twentieth aspect, it is possible to cause a vortex-ring-shaped air flow having high directivity to hit the target person (T).

A twenty-first aspect is the time notification device according to any one of the seventeenth to twentieth aspects in which the air flow discharging device (30) includes a component supplier (36) configured to add a predetermined discharge component to an air flow.

The twenty-first aspect is the time notification device in which the air flow discharging device (30) includes the component supplier (36) configured to add a predetermined discharge component to an air flow.

In the twenty-first aspect, it is possible to supply an air flow containing a predetermined discharge component to the target person (T).

### Brief Description of Drawings

[Fig. 1] Fig. 1 is a schematic overall diagram of a sleeper supporting device according to Embodiment 1.
[Fig. 2] Fig. 2 is a perspective view of an indoor space in which an air-flow generating unit is provided at a ceiling.
[Fig. 3] Fig. 3 is a perspective view of the air-flow generating unit.
[Fig. 4] Fig. 4 is a longitudinal sectional view of a vortex ring generator.
[Fig. 5] Fig. 5 is a cross-sectional view of the vortex ring generator.
[Fig. 6] Fig. 6 is a schematic overall diagram of a sleeper supporting device according to Modification 1.
[Fig. 7] Fig. 7 is a schematic overall diagram of a sleeper supporting device according to Modification 2.
[Fig. 8] Fig. 8 is a schematic overall diagram of a sleeper supporting device according to Modification 3.
[Fig. 9] Fig. 9 is a schematic overall diagram of a time notification device according to Embodiment 2.
[Fig. 10] Fig. 10 is a schematic block diagram of the time notification device according to Embodiment 2.
[Fig. 11] Fig. 11 is a schematic block diagram of a time notification device according to Modification 1 of Embodiment 2.
[Fig. 12] Fig. 12 is a schematic overall diagram of a time notification device according to Modification 2 of Embodiment 2.

### Description of Embodiments

Hereinafter, embodiments of the present disclosure will be described with reference to the drawings. The following embodiments are basically presented as preferred examples and do not intend to limit the present invention, applications thereof, or the range of use thereof.

### <<Embodiment 1>>

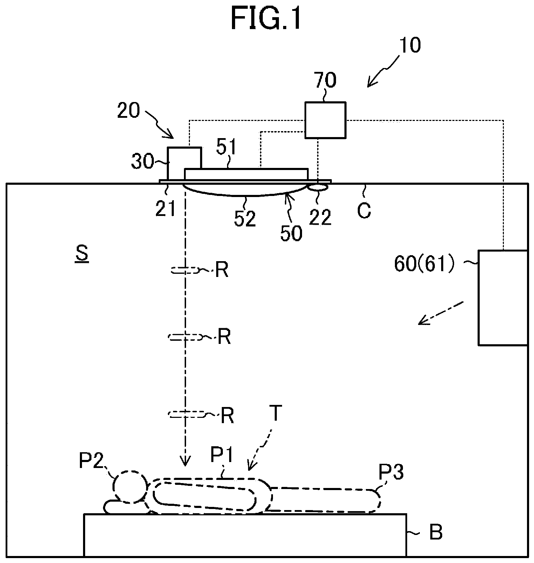

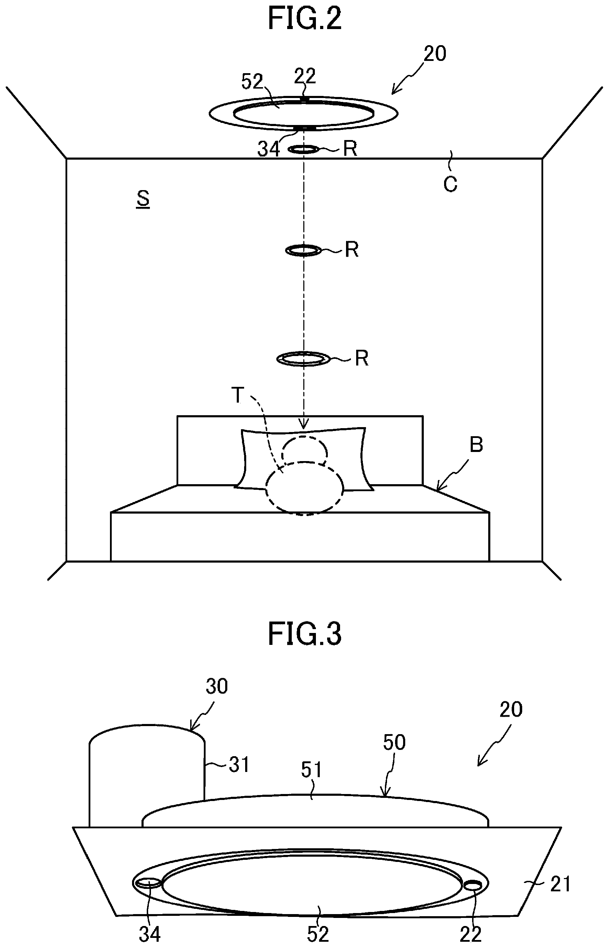

A sleeper supporting device (10) according to Embodiment 1 is a device that exerts a predetermined effect on a sleeper (T) that is a target person. As illustrated in Fig. 1 and Fig. 2, the sleeper (T) sleeps on bedding (B), such as a bed in, for example, an indoor space (S) that is a target space. The sleeper supporting device (10) includes an air-flow generating unit (20), an air conditioner (60), and a controller (70).

As illustrated in Fig. 1, the air conditioner (60) performs air conditioning of the indoor space (S). The air conditioner (60) has an indoor unit (61) of, for example, a wall-mount type. The indoor unit (61) is coupled to an outdoor unit (not illustrated) by a refrigerant pipe. The air conditioner (60) cools or heats indoor air by a refrigerant that performs a refrigeration cycle. The temperature of air in the indoor space (S) is thereby regulated. The air conditioner (60) may be able to regulate, in addition to the temperature of indoor air, humidity of the indoor air.

As illustrated in Fig. 1 and Fig. 3, the air-flow generating unit (20) has a fixing plate (21), an illumination device (50), and a vortex ring generator (30), and these are formed into a unit. The fixing plate (21) is formed to be a horizontal flat plate and placed at, for example, the back surface of a ceiling (C). The illumination device (50) includes an illumination body (51) and a cover (52) detachably attached to a lower portion of the illumination body (51). The illumination body (51) includes a light source, such as a LED, and a circuit board on which a light regulating circuit that regulates light of the light source is mounted. The cover (52) is constituted by a translucent resin material and exposed to the indoor space (S) .

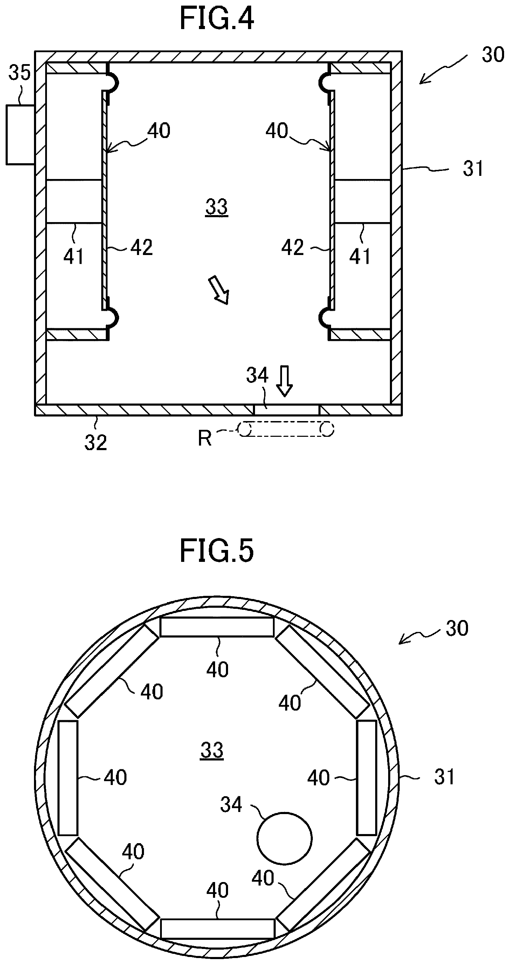

The vortex ring generator (30) constitutes an air flow discharging device that periodically or intermittently discharges an air flow. As illustrated in Fig. 4, the vortex ring generator (30) includes a cylindrical casing body (31) whose lower side is open and a lower cover (32) that closes the lower-side open face of the casing body (31). An air passage (33) is formed in an inner portion of the casing body (31). The lower cover (32) has a discharging port (34) in communication with the air passage (33). The discharging port (34) is open toward the indoor space (S). The discharging port (34) faces downward to be directed to the sleeper (T).

A plurality of extruding mechanisms (40) are provided in an inner portion of the casing body (31). The vortex ring generator (30) of the present example is provided with eight extruding mechanisms (40). The number of the extruding mechanisms (40) is merely presented as an example and may be one. Each of the extruding mechanisms (40) includes a linear actuator (41), which is a driver, and a vibrating plate (42) that is to be driven by the linear actuator (41). The linear actuator (41) displaces the vibrating plate (42) forward and backward. In the present example, the plurality of extruding mechanisms (40) are disposed along the peripheral wall of the casing body (31). The plurality of extruding mechanisms (40) are arranged at equal intervals in the circumferential direction.

The vortex ring generator (30) includes a drive control unit (35) for controlling the extruding mechanisms (40). The drive control unit (35) includes a circuit board connected to the linear actuator (41). The drive control unit (35) is disposed, for example, at an outer portion of the casing body (31) but may disposed in an inner portion of the casing body (31). The drive control unit (35) is configured to regulate the amplitude of vibrations of the vibrating plate (42) and the period of the vibrations.

Specifically, when the amplitude (displacement amount) of the vibrations of the vibrating plate (42) is regulated by the drive control unit (35), the velocity of an air flow (vortex ring (R)) discharged through the discharging port (34) is regulated. When the period of the vibrations of the vibrating plate (42) is regulated by the drive control unit (35), the period of the air flow (vortex ring (R)) discharged through the discharging port (34) is regulated. In other words, when the period of the vibrations of the vibrating plate (42) is regulated by the drive control unit (35), the number of times [cpm] (count per minute) of discharging an air flow through the discharging port (34), for example, per minute is regulated. The number of times [cpm] of discharging of an air flow is preferably set in a range of 40 or more and 90 or less. Thus, in the present embodiment, the period and the velocity of a discharged air flow are changed by the control of the extruding mechanisms (40). That is, the extruding mechanisms (40) constitute an air flow regulator that changes an air flow.

The air-flow generating unit (20) includes a Doppler sensor (22). The Doppler sensor (22) is attached to the ceiling (C) to be exposed to the indoor space (S). The Doppler sensor (22) uses micro waves to detect vibrations reflected by the sleeper (T). The Doppler sensor (22) detects a signal that serves as a base of biological information (heartbeat, respiration, large gross movement, and the like) of the sleeper (T). That is, the Doppler sensor (22) constitutes a biological information acquisition unit for acquiring biological information of the sleeper (T). In addition, the Doppler sensor (22) detects a signal that serves as a base of positional information such as the sleep position and the posture of the sleeper (T). That is, the Doppler sensor (22) is also used as a positional information acquisition unit for acquiring positional information of a sleeper.

The controller (70) is configured by using a microcomputer and a memory device (specifically, a semiconductor memory) that stores a software for causing the microcomputer to operate. The controller (70) is connected to the vortex ring generator (30), the illumination device (50), the air conditioner (60), and the Doppler sensor (22) in a wired or wireless manner and configured to be able to exchange a signal with these devices.

The controller (70) constitutes a control unit that controls the illumination device (50) and the air conditioner (60) in conjunction with the control of the vortex ring generator (30). In addition, the controller (70) outputs a predetermined instruction to the drive control unit (35) on the basis of a signal (biological information and positional information) detected by the Doppler sensor (22). The drive control unit (35) controls the extruding mechanisms (40) in accordance with the signal. As a result, the period/velocity of an air flow is changed on the basis of the signal detected by the Doppler sensor (22).

The controller (70) may be provided at the air-flow generating unit (20) and may be a body separated from the air-flow generating unit (20). For example, the controller (70) may be provided at a control unit or a remote controller of the air conditioner (60). The controller (70) may be provided at a server device connected to a local network, the internet, or the like, or at various types of communication terminals (a portable terminal, a personal computer, and the like).

### - Operation -

The operation of the sleeper supporting device (10) will be described with reference to Fig. 1 to Fig. 4.

### <Basic Operation of Vortex Ring Generator>

When the vortex ring generator (30) is operated, the vibrating plate (42) of each of the extruding mechanisms (40) is driven by the linear actuator (41). When the vibrating plate (42) vibrates forward and backward, air in the air passage (33) is extruded toward the discharging port (34). The air that passes through the discharging port (34) has a comparatively large velocity. In contrast, air at the periphery thereof is still. Therefore, at a discontinuity surface of the air in the air passage (33) and the air that passes through the discharging port (34), a shearing force is applied to the air, and a vortex flow is generated in the vicinity of an outer peripheral portion of the discharging port (34). The vortex flow forms an air flow (the vortex ring (R)) that moves downward from the discharging port (34). In Fig. 1, Fig. 2, and Fig. 4, the vortex ring (R) is schematically illustrated. The vortex ring (R) discharged through the discharging port (34) flows downward toward the sleeper (T) and hits a predetermined part of the sleeper (T). The vortex ring (R) of the present example hits a torso part (P1) of the sleeper (T).

The vortex ring (R) is discharged through the discharging port (34) of the vortex ring generator (30) periodically or intermittently in accordance with the vibration period of the vibrating plate (42). Accordingly, the vortex ring (R) hits the sleeper (T) periodically and intermittently. That is, an air flow hits the sleeper (T) at a predetermined rhythm. As described later in detail, as a result of a periodical air flow hitting the sleeper (T), a predetermined effect/efficacy relating to sleep can be obtained for the sleeper (T).

Here, the discharging port (34) of the vortex ring generator (30) is open to be directed to the sleeper (T). In addition, the vortex ring (R) has characteristics of having high directivity without easily diffusing. It is thus possible to reliably cause the vortex ring (R) to hit a predetermined part of the sleeper (T). Therefore, it is possible to reliably obtain a predetermined effect/efficacy for the sleeper (T).

The vortex ring generator (30) generates an air flow by the vibrations of the vibrating plate (42) and thus can discharge the vortex ring (R) of a comparatively short period. As a result, it is possible to cause an air flow of a comparatively short period to hit the sleeper (T).

### <Sleep Inducing Control>

During a predetermined period of time in which the sleeper (T) is caused to sleep, first control for inducing sleep of the sleeper (T) is performed. The first control is executed, for example, at a planned sleep time point previously set in a timer or the like.

In the first control, the vortex ring (R) of a comparatively long period is applied to the sleeper (T). The vortex ring (R) applies a periodical air pressure, for example, to the torso part (P1) (for example, the shoulders, the chest, and the like) of the sleeper (T). The periodical air pressure brings, to the sleeper (T), an effect similar to, for example, that of a mother periodically tapping the body of a child who intends to sleep. Application of such a periodical vortex ring (R) to the sleeper (T) can exert a sleep inducing effect on the sleeper (T) and induce comfortable sleep.

In the first control, the drive control unit (35) gradually lengthens the period of the vibrations of the vibrating plate (42). As a result, the period of discharging the vortex ring (R) is gradually lengthened, and the period of the vortex ring (R) that hits the sleeper (T) is also gradually lengthened. Consequently, it is possible to exert a sleep inducing effect on the sleeper (T) and induce comfortable sleep.

In addition, in the first control, the drive control unit (35) gradually decreases the amplitude of the vibrating plate (42). As a result, the velocity of the vortex ring (R) gradually decreases, and the air pressure of the vortex ring (R) that hits the sleeper (T) also gradually decreases. Consequently, it is possible to exert a sleep inducing effect on the sleeper (T) and induce comfortable sleep.

In the first control, control of the illumination device (50) is also performed by the controller (70). Specifically, the controller (70) gradually decreases the illuminance of the illumination device (50) in the first control. The illuminance in the indoor space (S) is thereby gradually decreased in conjunction with the above-described control of the vortex ring (R). Consequently, it is possible to exert a sleep inducing effect on the sleeper (T) and induce comfortable sleep. In addition, in the first control, control of the air conditioner (60) is also performed by the controller (70). In the first control, for example, a target temperature of indoor air is gradually decreased to gradually decrease the temperature of the indoor air.

### <Awakening Inducing Control>

During a predetermined period of time in which the sleeper (T) is caused to be awakened, second control for inducing awakening of the sleeper (T) is performed. The second control is executed at a planned wake-up time point previously set, for example, in a timer or the like.

In the second control, the vortex ring (R) of a comparatively short period is applied to the sleeper (T). Preferably, the period of the vortex ring (R) in the second control is shorter than the period of the vortex ring (R) in the first control. In the second control, the vortex ring (R) having a comparatively large velocity is applied to the sleeper (T). Preferably, the velocity of the vortex ring (R) in the second control is larger than the velocity of the vortex ring (R) in the first control. The vortex ring (R) periodically applies a comparatively large air pressure, for example, to the torso part (P1) of the sleeper (T). Consequently, it is possible to induce awakening of the sleeper (T) and induce the sleeper (T) to wake up comfortably.

In the second control, the drive control unit (35) gradually decreases the period of the vibrations of the vibrating plate (42). As a result, the period of discharging the vortex ring (R) gradually decreases, and the period of the vortex ring (R) that hits the sleeper (T) also gradually decreases. Consequently, it is possible to induce awakening of the sleeper (T) and induce the sleeper (T) to wake up comfortably.

In addition, in the second control, the drive control unit (35) gradually increases the amplitude of the vibrating plate (42). As a result, the velocity of the vortex ring (R) gradually increases, and the air pressure of the vortex ring (R) that hits the sleeper (T) also gradually increases. Consequently, it is possible to induce awakening of the sleeper (T) and induce the sleeper (T) to wake up comfortably.

In the second control, control of the illumination device (50) is also performed by the controller (70). Specifically, the controller (70) gradually increases the illuminance of the illumination device (50) in the second control. The illuminance in the indoor space (S) is thereby gradually increased in conjunction with the above-described change of the vortex ring (R). Consequently, it is possible to induce awakening of the sleeper (T) and induce the sleeper (T) to wake up comfortably. In addition, in the second control, control of the air conditioner (60) is also performed by the controller (70). In the second control, for example, a target temperature of indoor air is gradually increased to gradually increase the temperature of the indoor air.

In addition to the illuminance of the illumination device (50), the light color of the illumination device (50) may be regulated in the first control and the second control. In the first control and the second control, humidity of indoor air may be changed by the air conditioner (60) .

### <Control Based On Biological Information>

It is also possible to cause the vortex ring (R) at a predetermined rhythm to hit the sleeper (T), for example, during a sleep of the sleeper (T). Specifically, the heart rate of the sleeper (T) is detected by the Doppler sensor (22), and the vortex ring (R) at a rhythm that induces comfortable sleep of the sleeper (T) is applied to the sleeper (T) on the basis of the heart rate.

It is also possible to determine whether the sleeper (T) is in a REM sleep state or a non-REM sleep state on the basis of the heart rate. In this case, it is possible to cause the vortex ring (R) of a rhythm suitable for the sleeper (T) in the REM sleep state to hit the sleeper (T) and cause the vortex ring (R) of a rhythm suitable for the sleeper (T) in the non-REM sleep state to hit the sleeper (T) .

It is possible to detect a snore of the sleeper (T) on the basis of biological information detected by the Doppler sensor (22) and cause the vortex ring (R) of a rhythm suitable for suppressing the snore to hit the sleeper (T). As a snore detection unit other than the Doppler sensor (22), a microphone or the like that directly detects the acoustic pressure level at the periphery of the sleeper (T) is also usable.

### <Control Based On Positional Information>

It is possible to regulate the discharge period of the vortex ring (R) and the velocity of the vortex ring (R) on the basis of positional information of the sleeper (T) detected by the Doppler sensor (22). It is also possible to regulate the direction of the vortex ring (R) on the basis of such positional information (details will be described later).

### - Effects of Embodiment 1 -

The sleeper supporting device (10) according to Embodiment 1 includes the air flow discharging device (30) that has the discharging port (34) and that discharges an air flow intermittently through the discharging port (34) so as to cause the air flow to hit the sleeper (T) at a predetermined rhythm.

This configuration makes it possible to apply an air pressure of a predetermined rhythm to the sleeper (T) without providing a vibrator at the bedding (B) or the like, as in an example of the related art, and vibrating the bedding (B). It is thus possible to suppress, for example, vibrations from being transmitted to a different person in a room and a noise from being generated in the vicinity of the bedding (B). It is also possible to suppress the structure of the bedding (B) from becoming complex.

A predetermined effect relating to sleep can be exerted on the sleeper (T) by an air pressure. This effect includes an effect of inducing sleep of the sleeper (T), an effect of inducing awakening of the sleeper (T), an effect of improving comfortability of the sleep of the sleeper (T), and an effect of suppressing a snore of the sleeper (T).

It is possible to cause an air flow having directivity to hit the sleeper (T) through the discharging port (34) by causing the air flow to be generated through the discharging port (34). It is thus possible to reliably apply an air pressure to the sleeper (T) and to avoid an air flow from hitting a different person in a room. In addition, it is possible to cause an air flow to locally hit a predetermined part (for example, the torso part (P1)) of the sleeper (T).

The air flow discharging device (30) according to Embodiment 1 is a vortex ring generator having the extruding mechanisms (40) that carries air and in which air extruded by the extruding mechanisms (40) is discharged as a vortex-ring-shaped air flow through the discharging port (34).

This configuration makes it possible to cause the vortex ring (R) discharged through the discharging port (34) to hit the sleeper (T) at a predetermined rhythm. The air of the vortex ring (R) does not easily diffuse and has high directivity, and thus can reliably apply an air pressure to the sleeper (T).

The extruding mechanisms (40) according to Embodiment 1 each include the linear actuator (41) (driver) and the vibrating plate (42) to be driven by the linear actuator (41) .

This configuration makes it possible to generate the vortex ring (R) at a comparatively short period by vibrating the vibrating plate (42). It is thus possible to cause the vortex ring (R) to hit the sleeper (T) frequently and to induce a predetermined effect relating to sleep.

The sleeper supporting device (10) according to Embodiment 1 includes the extruding mechanisms (40) (air flow regulator) that change an air flow. Specifically, the extruding mechanisms (40) regulate the period of discharging an air flow. In addition, the extruding mechanisms (40) regulate the velocity of an air flow.

Various effects can be exerted on the sleeper (T) by changing the period of discharging an air flow. Sleep of the sleeper (T) can be induced by gradually lengthening the period of discharging an air flow. Awakening of the sleeper (T) can be induced by gradually shortening the period of discharging an air flow.

The extruding mechanisms (40) can easily change the period of discharging an air flow by regulating the period of the vibrations of the vibrating plate (42).

Various effects can be exerted on the sleeper (T) by changing the velocity of discharging an air flow. Sleep of the sleeper (T) can be induced by gradually decreasing the velocity of discharging an air flow. Awakening of the sleeper (T) can be induced by gradually lengthening the period of discharging an air flow.

The extruding mechanisms (40) can easily change the velocity of discharging an air flow by regulating the amplitude or the displacement amount of the vibrations of the vibrating plate (42).

The sleeper supporting device (10) according to Embodiment 1 includes the Doppler sensor (22) (biological information acquisition unit) that acquires biological information of the sleeper (T), and an air flow regulator (40) changes an air flow on the basis of the biological information acquired by the Doppler sensor (22).

This configuration makes it possible to cause an air flow whose period, velocity, and the like are optimized in accordance with biological information, such as heartbeat, respiration, and snores, of the sleeper (T) to hit the sleeper (T).

The sleeper supporting device (10) according to Embodiment 1 includes the Doppler sensor (22) (positional information acquisition unit (22)) that acquires positional information of the sleeper (T), and the extruding mechanisms (air flow regulator (40)) change an air flow on the basis of the positional information of the sleeper (T) acquired by the positional information acquisition unit (22).

This configuration makes it possible to cause an air flow whose period, velocity, and the like are optimized in accordance with the position, the posture, and the like of the sleeper (T) to hit the sleeper (T).

The Doppler sensor (22) is used as both the biological information acquisition unit and the positional information acquisition unit and thus can reduce the number of parts.

The vortex ring generator (30) (air flow discharging device) according to Embodiment 1 discharges an air flow during a period of time in which the sleeper (T) is caused to sleep.

This configuration makes it possible to induce sleep of the sleeper (T) by an air flow of a predetermined rhythm.

The air flow discharging device (30) according to the embodiment discharges the air flow at the time of wake-up during the period of time in which the sleeper (T) is caused to be awakened.

This configuration makes it possible to induce awakening of the sleeper (T) by an air flow of a predetermined rhythm.

The vortex ring generator (30) (air flow discharging device) according to the embodiment is installed at the ceiling (C). By installing the vortex ring generator (30) at the ceiling (C), it becomes easy to cause an air flow to hit the sleeper (T).

### <<Modifications of Embodiment 1>>

The above-described Embodiment 1 may have the configurations of the following modifications. The configurations to be described with the following modifications are also applicable to Embodiment 2, for which details will be described later.

### <Modification 1>

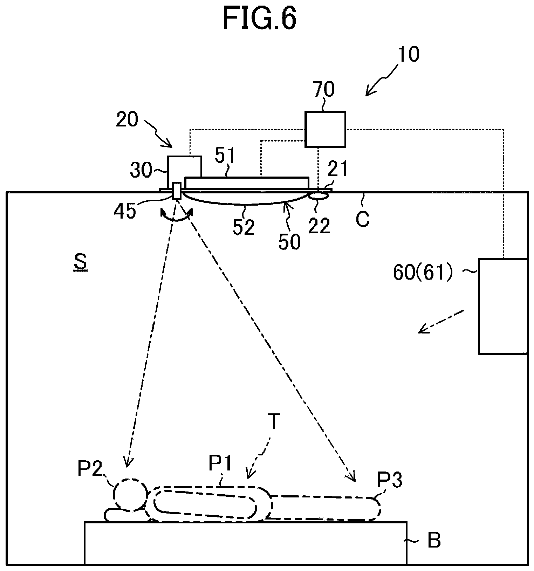

In Modification 1 illustrated in Fig. 6, the vortex ring generator (30) is provided with a movable nozzle (45). The movable nozzle (45) is coupled to a motor via a rotary shaft. The discharging port (34) in communication with the air passage (33) is formed at the tip (lower end) of the movable nozzle (45). When the rotary shaft is driven to rotate by the motor, the direction of the movable nozzle (45) is regulated, and the direction of the discharging port (34) is thereby changed. That is, the movable nozzle (45) of the present example constitutes an air flow regulator that regulates the direction of an air flow.

The controller (70) controls the direction of the movable nozzle (45) on the basis of the positional information of the sleeper (T) detected by the Doppler sensor (22). For example, when the position of the sleeper (T) on the bedding (B) is largely displaced, the direction of the discharging port (34) of the movable nozzle (45) is regulated to follow the sleeper (T). Consequently, it is possible to reliably cause the vortex ring (R) to hit a predetermined part of the sleeper (T).

Moreover, by changing the direction of the movable nozzle (45), a part of the sleeper (T) that the vortex ring (R) is caused to hit can be optionally changed. As illustrated in Fig. 6, the direction of the movable nozzle (45) can be regulated, for example, to aim at a head part (P2) of the sleeper (T) or a foot part (P3) of the sleeper (T) .

### <Modification 2>

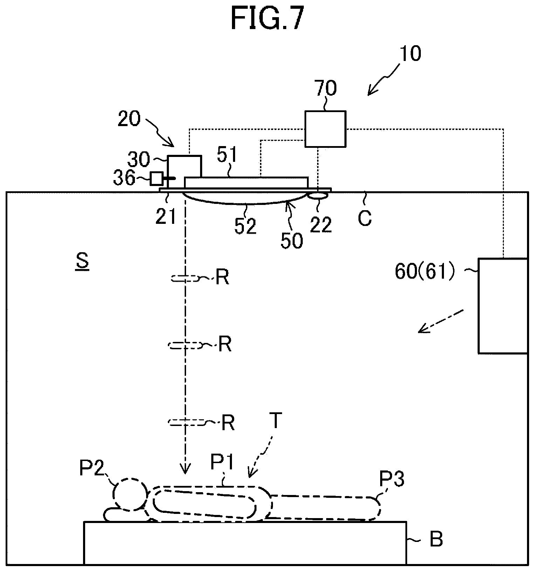

In Modification 2 illustrated in Fig. 7, the vortex ring generator (30) is provided with a component supplier (36). The component supplier (36) is a device for adding a predetermined discharge component to an air flow (vortex ring (R)). The discharge component is, for example, a fragrance component, a functional component having predetermined efficacy, water, oxygen, carbon dioxide, and the like. The discharge component may not be necessarily a gas and may be a fine liquid. The component supplier (36), for example, vaporizes a material of the discharge component and then supplies the vaporized component to the air passage (33). Consequently, the discharge component is also contained in the vortex ring (R) discharged through the discharging port (34).

In the above-described first control for inducing sleep of the sleeper (T), a fragrance component or a functional component that induces sleep of the sleeper (T) is added into the vortex ring (R). In the first control, oxygen may be added into the vortex ring (R). Consequently, sleep of the sleeper (T) can be induced. In the above-described second control for inducing awakening of the sleeper (T), a fragrance component or a functional component that induces awakening of the sleeper (T) is added into the vortex ring (R). Consequently, awakening of the sleeper (T) can be induced.

### <Modification 3>

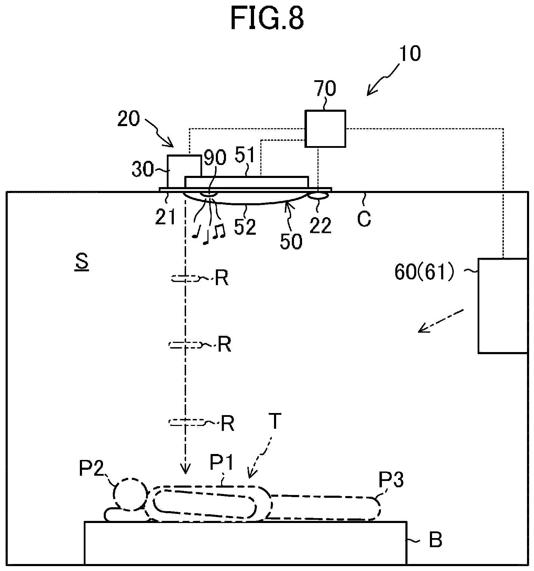

The sleeper supporting device (10) according to Modification 3 illustrated in Fig. 8 is provided with a sound generator that generates a predetermined sound in the indoor space (S). The sound generator of the present example is constituted by a speaker (90) that is to be mounted at the air-flow generating unit (20). The controller (70) controls the speaker (90) in conjunction with the control of the vortex ring generator (30) and causes the speaker (90) to generate a predetermined sound. As this sound, for example, a natural sound is used.

In the above-described first control for inducing sleep of the sleeper (T), a sound that induces sleep of the sleeper (T) is output from the speaker (90). Consequently, sleep of the sleeper (T) can be induced. In the above-described second control for inducing awakening of the sleeper (T), a sound that induces awakening of the sleeper (T) is output. Consequently, awakening of the sleeper (T) can be induced.

A predetermined sound may be generated by vibrating the vibrating plate (42) of the vortex ring generator (30). In this case, the vortex ring generator (30) is also used as a sound generator and thus can reduce the number of parts.

A predetermined sound may be generated by vibrating the cover (52) of the illumination device (50). In this case, the illumination device (50) is also used as a sound generator and thus can reduce the number of parts. In addition, a generated sound can be reliably transmitted to the sleeper (T).

### <<Embodiment 2>>

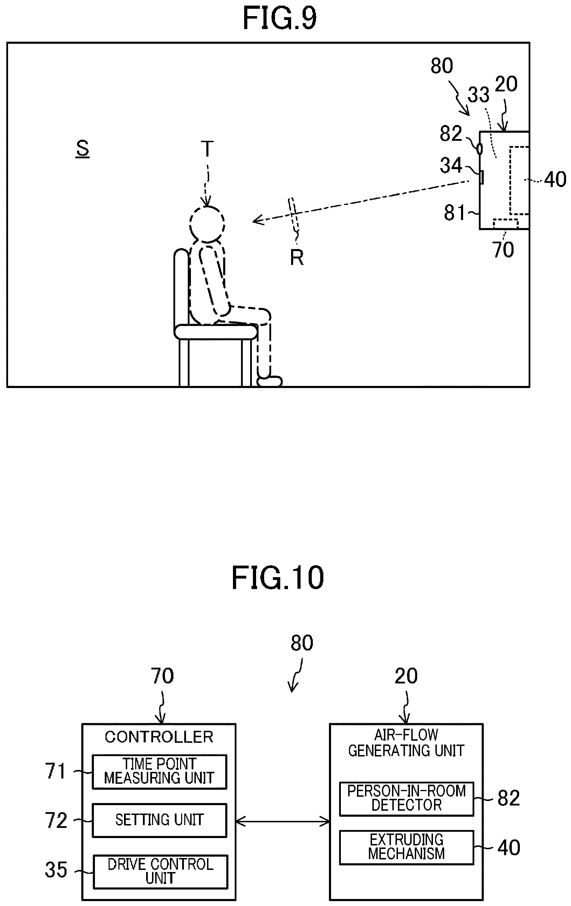

A time notification device (80) according to Embodiment 2 is a device that exerts a time notification effect on a target person (T). The "time" mentioned here includes "time point" and "period of time that has elapsed". As illustrated in Fig. 9, the target person (T) is present in the indoor space (S) that is a target space. The target person (T) sits, for example, on a chair in the indoor space (S). The time notification device (80) includes the vortex ring generator (30), which is an air flow discharging device, and the controller (70).

The vortex ring generator (30) has a casing (81) and the extruding mechanism (40) disposed in an inner portion of the casing (81).

The casing (81) is mounted at a wall surface in the indoor space (S). In other words, the vortex ring generator (30) is of a wall-mount type. The air passage (33) is formed in an inner portion of the casing (81). The discharging port (34) is formed at the front surface of the casing (81). The discharging port (34) is open toward the indoor space (S). An air flow (vortex ring (R)) is discharged toward the indoor space (S) through the discharging port (34).

The extruding mechanism (40) has the same configuration as that in Embodiment 1. The extruding mechanism (40) has a linear actuator and a vibrating plate that is to be driven by the linear actuator (not illustrated).

The vortex ring generator (30) is provided with a person-in-room detector (82) that detects presence of the target person (T) in a target space (S). The person-in-room detector (82) is constituted by the above-described Doppler sensor, an infrared sensor, or the like.

The controller (70) is configured by using a microcomputer and a memory device (specifically, a semiconductor memory) that stores a software for causing the microcomputer to operate. The controller (70) is connected to the vortex ring generator (30) in a wired or wireless manner. The controller (70) is configured to be able to exchange a signal with the extruding mechanism (40) and the person-in-room detector (82). The controller (70) of the present example is provided at the vortex ring generator (30). The controller (70) may be a body separated from the vortex ring generator (30). The controller (70) may be provided at a server device connected to a local network, the internet, or the like, or at various types of communication terminals (a portable terminal, a personal computer, and the like).

As illustrated in Fig. 10, the controller (70) has a time point measuring unit (71), a setting unit (72), and the drive control unit (35). The time point measuring unit (71) measures a current time point incrementally. A time point previously desired by a user or the like is set in the setting unit (72). The number of time points set in the setting unit (72) may be one per day or may be more than one per day.

The drive control unit (35) controls the extruding mechanism (40). When a first condition is established, the drive control unit (35) of the present example controls the extruding mechanism (40) to discharge an air flow through the discharging port (34). The first condition includes the following conditions a and b.

Condition a: a current time point measured by the time point measuring unit (71) reaches a time point set in the setting unit (72).

Condition b: the target person (T) in the target space (S) is detected by the person-in-room detector (82).

When both of the condition a and the condition b are established, the drive control unit (35) drives the extruding mechanism (40). Consequently, the vortex ring generator (30) performs a discharging operation at every time when a current time point reaches a set time point. The discharging operation corresponds to a first operation. In the discharging operation, the vortex ring generator (30) discharges an air flow through the discharging port (34) so that an air flow for notifying the target person (T) of a time (strictly, a time point) hits the target person (T). The discharged air flow hits the target person (T) in the indoor space (S). The time notification device (80) may have a configuration in which, without the person-in-room detector (82), the discharging operation is performed when only the condition a is established.

The vortex ring generator (30) performs the discharging operation periodically at every set time point set in the setting unit (72). The discharging operation corresponds to the first operation. In the discharging operation, the vortex ring generator (30) discharges an air flow through the discharging port (34) so that an air flow for notifying the target person (T) of a time (strictly, a time point) to hit the target person (T).

The number of air flows (that is, the vortex rings (R)) to be discharged through the discharging port (34) in one discharging operation is changeable in accordance with the setting of the setting unit (72). A user or the like can set a first mode and a second mode selectively in the setting unit (72).

In the first mode, a user can optionally set a number N of the vortex rings (R) to be discharged through the discharging port (34). Thus, in the discharging operation in the first mode, the vortex ring (R) is discharged N time through the discharging port (34) when a current time point reaches a set time point.

In the second mode, the controller (70) determines the number N of the vortex rings (R) to be discharged through the discharging port (34), in accordance with a set time point. For example, when the set time point is H hour M minute, N equals H. Specifically, a set time point set in the setting unit (72) is, for example, 3 hours 0 minutes. In this case, when the current time point reaches 3 hours 0 minutes, the controller (70) controls the vortex ring generator (30) to discharge the vortex ring (R) three times through the discharging port (34). In the discharging operation in the second mode, the vortex rings (R) of a number corresponding to a current time point or a set time point hit the target person (T). Accordingly, the target person (T) can know the current time point on the basis of the number of the vortex rings (R) that have hit the target person (T).

### - Effects of Embodiment 2 -

In Embodiment 2, the air flow discharging device (30) performs the first operation in which an air flow is intermittently discharged through the discharging port (34) so that an air flow for notifying the target person (T) of a time to hit the target person (T) periodically.

This configuration makes it possible to notify the target person (T) of a time without vibrations being transmitted to the periphery of the target person (T). The target person (T) can know a time even when the target person (T) has a visual disorder.

It is possible to cause an air flow having directivity to hit the target person (T) through the discharging port (34) by generating the air flow through the discharging port (34). It is thus possible to reliably apply an air pressure to the target person (T).

In Embodiment 2, the first operation is performed at every set time point.

This configuration enables the target person (T) to know that a current time point has reached a set time point.

In Embodiment 2, the air flow discharging device (30) discharges air flows of a number corresponding to a current time point through the discharging port (34) in the first operation.

This configuration enables the target person (T) to specifically know a current time point on the basis of the number of discharged air flows.

In Embodiment 2, the air flow discharging device (30) is a vortex ring generator having the extruding mechanism (40) that carries air, and in which air extruded by the extruding mechanism (40) is discharged as a vortex-ring-shaped air flow through the discharging port (34).

This configuration makes it possible to cause the vortex ring (R) discharged through the discharging port (34) to hit the target person (T). The air of the vortex ring (R) does not easily diffuse and has high directivity, and thus can reliably apply an air pressure to the target person (T).

### <<Modifications of Embodiment 2>>

Embodiment 2 described above may have the configurations of the following modifications.

### <Modification 1>

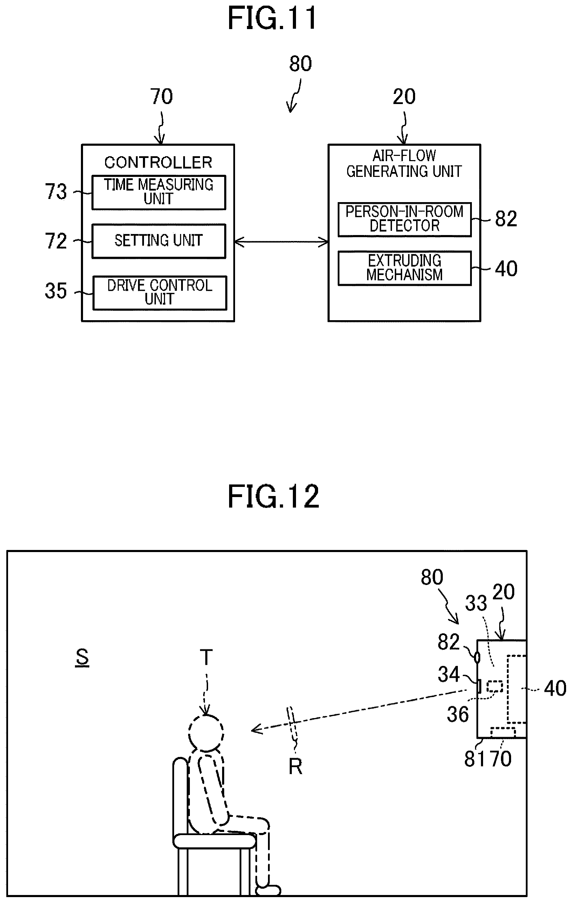

As illustrated in Fig. 11, the time notification device (80) of Modification 1 of Embodiment 2 differs from that of Embodiment 2 in terms of the configuration of the controller (70). The controller (70) is provided with a time measuring unit (73), instead of the time point measuring unit (71). An elapsed time desired by a user or the like is set in the setting unit (72). The time measuring unit (73) counts time until the time set in the setting unit (72) has elapsed. When the time measured by the time measuring unit (73) is counted up, the controller (70) controls the vortex ring generator (30) to cause the vortex ring generator (30) to perform the first operation. In other words, the controller (70) controls the vortex ring generator (30) to perform a discharging operation at every time set in the setting unit (72). An air flow discharged through the discharging port (34) thus hits the target person (T) periodically at every set time. Consequently, the target person (T) can know, as appropriate, a lapse of the time set in the setting unit (72) .

### <Modification 2>

As illustrated in Fig. 12, the time notification device (80) of Modification 2 of Embodiment 2 is provided with the component supplier (36) described in Embodiment 1. The component supplier (36) is provided at the vortex ring generator (30). The component supplier (36) is disposed at the air passage (33) in the casing (81). The component supplier (36) is a device for adding a predetermined discharge component to an air flow (vortex ring (R)). The discharge component may be a functional component having predetermined efficacy, water, oxygen, carbon dioxide, or the like. The discharge component in the present modification is a fragrance component. The discharge component may not be necessarily a gas and may be a fine liquid. The component supplier (36), for example, vaporizes a material of the discharge component and then supplies the vaporized component to the air passage (33). Consequently, the discharge component is also contained in the vortex ring (R) discharged through the discharging port (34).

In the discharging operation of the vortex ring generator (30), the component supplier (36) adds a fragrance component to the air in the air passage (33). In the discharging operation, an air flow containing the fragrance component hits the target person (T). The target person (T) thus can know a current time point or an elapsed time by using not only a tactile sense but also an olfactory sense. The target person (T) thus can reliably know a current time point or an elapsed time.

### <Modification 3>

As with Modification 1 of Embodiment 1, the time notification device (80) of Modification 3 of Embodiment 2 is provided with the movable nozzle (45), which is an air flow regulator, and the positional information acquisition unit. The positional information acquisition unit is constituted by, for example, the Doppler sensor (22). The Doppler sensor (22) acquires positional information of the target person (T). On the basis of the positional information acquired by the Doppler sensor (22), the controller (70) regulates the direction of the discharging port (34) of the movable nozzle (45) so as to follow the target person (T). Consequently, in the discharging operation, an air flow discharged through the discharging port (34) easily hits the target person (T).

### <<Other Embodiments>>

Embodiment 1, Embodiment 2, and modifications of these Embodiments may be configured as follows.

The sleeper supporting device (10) may be constituted by only the air flow discharging device (30).

The air flow discharging device (30) may not be necessarily the vortex ring generator (30) and may have any configuration as long as the air flow discharging device (30) discharges an air flow through the discharging port (34) periodically.

The extruding mechanism (40) may not be necessary configured to have the driver (41) and the vibrating plate (42). For example, the extruding mechanism (40) may be a piston that reciprocates in the air passage (33) to carry air.

The air flow discharging device (vortex ring generator (30)) according to the above-described Embodiment 1 is installed at a ceiling. The air flow discharging device (30) according to Embodiment 1, however, may be of a floor-installation type to be installed at a floor surface or may be a wall-mount type to be installed at a wall surface. The air flow discharging device (30) according to Embodiment 1 also may be installed on a furniture in a room and may be preferably installed on a table or a desk.

The air flow discharging device (30) according to the above-described Embodiment 2 is installed at a wall surface. The air flow discharging device (30) according to Embodiment 2, however, may be installed at a ceiling or at a floor surface. The air flow discharging device (30) according to Embodiment 2 may be installed on a furniture in a room and may be preferably installed on a table or a desk.

The air flow discharging device (30) may simply cause an air flow to hit the sleeper (T). For example, the air flow discharging device (30) may be mounted on a baby carriage. In this case, the air flow discharging device discharges an air flow toward an infant, which is a target person.

The biological information acquisition unit may not be necessarily the Doppler sensor (22). The biological information acquisition unit may be a piezoelectric element that directly detects a body movement of the sleeper (T) or a pneumatic sensor that detects a change in an air pressure in response to a change in a body movement of the sleeper (T) .

The positional information acquisition unit may not be necessarily the Doppler sensor (22). The positional information acquisition unit may be an infrared sensor or a gyro sensor.

The controller (70) may control a ventilator that ventilates a room in conjunction with the air flow discharging device (30). For example, in the first control, the controller (70) controls the ventilator so as to decrease a ventilation amount by a certain degree. The concentration of carbon dioxide in the indoor space (S) is thereby slightly decreased, which induces sleep of the sleeper (T).

The air flow discharging device (30) may be applied to a device that notifies a person of a rhythm of a short period. Specifically, the air flow discharging device (30) may be used for a metronome. In this configuration, the air flow discharging device (30) is preferably the above-described vortex ring generator. A vortex ring is intermittently generated through the discharging port (34) of the air flow discharging device (30), the vortex ring hits the target person (T), and the target person (T) can thereby grasp a rhythm of a short period.

The air flow discharging device (30) may be a relief giving device that exerts an effect of giving a sense of relief on the target person (T). In this configuration, the air flow discharging device (30) is preferably the above-described vortex ring generator. An air flow (vortex ring) is intermittently discharged through the discharging port (34) of the air flow discharging device (30) to cause the vortex ring to hit the target person (T). When a vortex ring periodically hits the shoulders and the like of the target person (T), the target person (T) feels a sense of relief. The air flow discharging device (30) preferably discharges a vortex ring of a predetermined rhythm effective for obtaining a relieving effect.

Although embodiments and modifications have been described above, it should be understood that various changes are possible in the forms and the details without departing from the gist and the scope of the claims. The embodiments, the modifications, and the other embodiments described above may be combined together or replaced, as appropriate, as long as functions of an object of the present disclosure are involved. The wordings such as "first", "second", "third", and so on mentioned above are used to distinguish words to which these wordings are added from each other and do not intend to limit the number and even the order of the words.

### Industrial Applicability

The present disclosure is useful for an air flow discharging device, a sleeper supporting device, and a time notification device.

### Reference Signs List

- 10: sleeper supporting device
- 22: positional information acquisition unit
- 22: biological information acquisition unit
- 30: air flow discharging device
- 34: discharging port
- 36: component supplier
- 40: extruding mechanism (air flow regulator)
- 41: linear actuator (driver)
- 42: vibrating plate
- 45: movable nozzle (air flow regulator)
- 50: illumination device
- 70: controller (control unit)
- 80: time notification device
- 90: sound generator

## Claims

1. An air flow discharging device comprising:
a discharging port (34),
wherein the air flow discharging device is configured to intermittently discharge an air flow through the discharging port (34) so that an air flow that exerts a predetermined effect hits a target person (T) periodically.

2. A sleeper supporting device comprising:
the air flow discharging device (30) according to claim 1,
wherein the air flow discharging device (30) is configured to intermittently discharge an air flow through the discharging port (34) so that an air flow hits a sleeper (T) that is the target person at a predetermined rhythm.

3. The sleeper supporting device according to claim 2,
wherein the air flow discharging device (30) is a vortex ring generator that has an extruding mechanism (40) configured to carry air, and that is configured such that air extruded by the extruding mechanism (40) is discharged as a vortex-ring-shaped air flow through the discharging port (34).

4. The sleeper supporting device according to claim 3,
wherein the extruding mechanism (40) has a driver (41) and a vibrating plate (42) configured to be driven by the driver (41).

5. The sleeper supporting device according to any one of claims 2 to 4, further comprising:
an air flow regulator (40, 45) configured to change the air flow.

6. The sleeper supporting device according to claim 5,
wherein the air flow regulator (40) is configured to regulate a period of discharging an air flow.

7. The sleeper supporting device according to claim 5 or claim 6,
wherein the air flow regulator (40) is configured to regulate a velocity of an air flow.

8. The sleeper supporting device according to any one of claims 5 to 7,
wherein the air flow regulator (45) is configured to regulate a direction of an air flow.

9. The sleeper supporting device according to any one of claims 5 to 8, further comprising:
a biological information acquisition unit (22) configured to acquire biological information of the sleeper (T),
wherein the air flow regulator (40) is configured to change the air flow based on biological information acquired by the biological information acquisition unit (22).

10. The sleeper supporting device according to any one of claims 5 to 9, further comprising:
a positional information acquisition unit (22) configured to acquire positional information of the sleeper (T),
wherein the air flow regulator (40) is configured to change the air flow based on positional information of the sleeper (T) acquired by the positional information acquisition unit (22).

11. The sleeper supporting device according to any one of claims 2 to 10,
wherein the air flow discharging device (30) is configured to discharge the air flow during a period of time in which the sleeper (T) is caused to sleep.

12. The sleeper supporting device according to any one of claims 2 to 11,
wherein the air flow discharging device (30) is configured to discharge the air flow at a time of wake-up during a period of time in which the sleeper (T) is caused to be awakened.

13. The sleeper supporting device according to any one of claims 2 to 12, further comprising:
an illumination device (50) configured to illuminate a target space (S) in which the sleeper is present, and
a control unit (70) configured to control the illumination device (50) in conjunction with the air flow discharging device (30).

14. The sleeper supporting device according to any one of claims 2 to 13, further comprising:
a sound generator (90) configured to transmit a sound to a target space (S) in which the sleeper (T) is present; and
a control unit (70) configured to control the sound generator (90) in conjunction with the air flow discharging device (30).

15. The sleeper supporting device according to any one of claims 2 to 14,
wherein the air flow discharging device (30) includes a component supplier (36) configured to add a predetermined discharge component to an air flow.

16. The sleeper supporting device according to any one of claims 2 to 15,
wherein the air flow discharging device (30) is configured to be installed at a ceiling (C).

17. A time notification device comprising:
the air flow discharging device (30) according to claim 1,
wherein the air flow discharging device (30) is configured to perform a first operation of discharging an air flow through the discharging port (34) so that an air flow for notifying the target person (T) of a time hits the target person (T) periodically.

18. The time notification device according to claim 17,
wherein the air flow discharging device (30) is configured to perform the first operation at every set time interval or every set time point.

19. The time notification device according to claim 17 or claim 18,
wherein the air flow discharging device (30) is configured to discharge air flows of a number corresponding to a current time point through the discharging port (34) in the first operation.

20. The time notification device according to any one of claims 17 and 19,
wherein the air flow discharging device (30) is a vortex ring generator that has an extruding mechanism (40) configured to carry air, and that is configured such that air extruded by the extruding mechanism (40) is discharged as a vortex-ring-shaped air flow through the discharging port (34).

21. The time notification device according to any one of claims 17 to 20,
wherein the air flow discharging device (30) includes a component supplier (36) configured to add a predetermined discharge component to an air flow.
